# EUROPÄISCHE PATENTSCHRIFT

(11) **EP 1 361 830 B1**
(45) Veröffentlichungstag und Bekanntmachung des Hinweises auf die Patenterteilung: **26.05.2004**
(21) Anmeldenummer: 01985420.7
(22) Anmeldetag: 18.12.2001
(51) Int. Cl.: A61C 1/14, A61C 3/03, A61B 17/16

(54) **MEDIZINISCHES ODER DENTALMEDIZINISCHES HANDSTÜCK UND EIN WERKZEUG FÜR DAS HANDSTÜCK**
MEDICAL OR DENTAL HANDPIECE AND A TOOL FOR SAID HANDPIECE
PIECE A MAIN MEDICALE OU DENTAIRE ET OUTIL DESTINE A CETTE PIECE A MAIN

(30) Priorität: 22.02.2001 DE 20103179 U
(43) Veröffentlichungstag der Anmeldung: 19.11.2003
(73) Patentinhaber: Kaltenbach & Voigt GmbH & Co. KG, 88400 Biberach/Riss (DE)
(72) Erfinder: KRAMER, Markus, 88400 Biberach (DE); HOFER, Bruno, 88416 Erlenmoos (DE)
(74) Vertreter: Schmidt-Evers, Jürgen, Dipl.-Ing.
(86) Internationale Anmeldenummer: PCT/EP2001/014925
(87) Internationale Veröffentlichungsnummer: WO 2002/069829

(56) Entgegenhaltungen:
- DE-A- 3 930 645
- US-A- 5 505 617

## Beschreibung

Die Erfindung bezieht sich auf medizinisches oder dentalmedizinisches Handstück nach dem Oberbegriff des Anspruchs 1 und ein Werkzeug für das Handstück nach dem Oberbegriff des Anspruchs 2.

Ein Handstück und ein Werkzeug dieser Arten sind in der DE 42 18 683 A1 beschrieben. Das Werkzeug ist bei dieser vorbekannten Ausgestaltung mit einem Halteschaft in eine Aufnahmehülse des Handstücks einsteckbar und lösbar fixierbar. Die Aufnahmehülse ist durch einen im Handstück angeordneten Antrieb in ihrer Achsrichtung hin und her verschiebbar im Handstück gelagert. Zur Fixierung des Halteschaftes in der Aufnahmehülse ist eine Fixiervorrichtung vorgesehen, die den Schaft durch Klemmwirkung gegen eine unbeabsichtigte Bewegung in der Aufnahmehülse sichern soll. Der Halteschaft kann im Querschnitt z. B. C-förmig ausgebildet sein, wobei die Klemmwirkung durch eine elastische Spreizspannung der C-Form erreicht wird. Die Aufnahmehülse ist bei Gewährleistung ihrer axialen Verschiebbarkeit drehbar im Handstück gelagert und durch eine manuell überdrückbare ringförmige Zahnkupplung in Umfangsrichtung positioniert. Es ist somit möglich, durch ein von außen zugängliches Drehangriffsglied die Drehkupplung zu überdrücken und die Aufnahmehülse in eine wahlweise Drehstellung zu drehen, in der sie jeweils wieder positioniert ist. Bei dieser bekannten Ausgestaltung hat es sich in der Praxis gezeigt, daß die Fixiervorrichtung bei in der Achsrichtung der Aufnahmehülse wirksamen Belastungen unbeabsichtigt nachgeben kann, was auf der schwierig kontrollierbaren Klemmwirkung beruht.

Aus DE 39 30 645 A1 ist ein zahnärztliches Instrument zu entnehmen, mit einer durch einen Drehantrieb drehbaren Aufnahmehülse für ein Werkzeug, das einen längs geschlitzten zylindrischen Halteschaft aufweist, mit dem es in eine Aufnahmebohrung der Aufnahmehülse einsteckbar ist. Zur Drehsicherung des Werkzeugs in der Aufnahmehülse sind am Öffnungsrand der Aufnahmehülse auf den Umfang verteilt angeordnete Ausnehmungen vorgesehen, in die wenigstens ein Nocken im Fußbereich des Halteschaftes einsteckbar ist. Zur axialen Sicherung des Werkzeugs dienen zwei Verrastungsnasen, die im freien Endbereich von zwei durch die Schlitzung des Halteschaftes quer einander gegenüberliegend angeordneten Federarmen angeordnet sind und im eingesteckten Zustand in einen weiteren Teil 56 der Bohrung 54 (siehe Spalte 6, Absatz 3) einfassen und somit eine ringförmige Hinterschneidung hintergreifen.

Das Handstück und das Werkzeug gemäß DE 3 930 645 A1 sind von den in den Oberbegriffen der neven Ansprüche 1 und 2 angegebenen Arten und somit gottungsgemäß.

Der Erfindung liegt deshalb die Aufgabe zugrunde, bei einem Handstück und bei einem Werkzeug der eingangs angegebenen Arten wenigstens die axiale Fixierwirkung zu verbessern.

Diese Aufgabe wird durch die Merkmale des Anspruchs 1 bzw. 2 gelöst. Vorteilhafte Weiterbildungen der Erfindung sind in den Unteransprüchen beschrieben.

Bei der erfindungsgemäßen Ausgestaltung weist der Halteschaft des Werkzeugs wenigstens eine radial elastisch einfederbare Verrastungsnase auf, die einen sich in der Umfangsrichtung erstreckenden Rand der Hinterschneidung hintergreift. Die Rückenfläche der Verrastungsnase ist vorzugsweise durch eine Einführungsfläche gebildet, aufgrund der die Verrastungsnase beim Einstecken in die Aufnahmehülse selbsttätig einfedert.

Bei der erfindungsgemäßen Ausgestaltung ist das Werkzeug in seiner axialen Funktionsstellung in der Aufnahmehülse lösbar verrastbar und somit gegen eine unbeabsichtigte axiale Verschiebung gesichert. Um diese Sicherungsfunktion zu erfüllen, bedarf es grundsätzlich nur einer radial elastisch einfederbaren Verrastungsnase. Vorzugsweise sind wenigstens zwei auf den Umfang verteilt angeordnete Verrastungsnasen vorgesehen, die die axiale Fixierung verstärken und eine gleichmäßige Verteilung der Verrastungskraft gewährleisten. Gegen eine unbeabsichtigte Verdrehung kann der Halteschaft in der Aufnahmehülse dadurch gesichert sein daß er mit einer elastischen Klemmspannung radial nach außen gegen die Wandung der Aufnahmehülse drückt.

Bei der Einführungsfläche kann es sich um eine konvex gerundete oder schräge Einführungsfläche handeln. Vorzugsweise handelt es sich um eine kegelförmige Einführungsfläche, die bei linienförmiger Anlage an der Aufnahmehülse in der Einführungsphase eine geringe Materialbeanspruchung und einen geringen Verschleiß ermöglicht.

Im Rahmen der Erfindung kann die Hinterschneidung an der Aufnahmehülse durch deren inneres freies Ende gebildet sein, wobei der Innenumfangsrand der Aufnahmehülse eine Verrastungskante bzw. -fläche bildet und das Werkzeug in jeder wahlweisen Drehposition verrastbar ist. Hierbei kann das Werkzeug in der Aufnahmehülse frei drehbar oder schwergängig drehbar gelagert sein, wodurch es sich in wahlweise Drehstellungen einstellen läßt oder im Funktionsbetrieb selbsttätig anpaßt. Wenn die Hinterschneidung durch einen radialen vorzugsweise durchgehenden Schlitz in der Innenumfangswand der Aufnahmehülse gebildet ist, ergibt sich zugleich eine Drehsicherung für das Werkzeug dadurch, daß die Verrastungsnase in den Schlitz einfaßt. Die Seitenränder des Schlitzes bilden hierbei die Verrastungsnase seitlich begrenzende Anschläge, die ein Drehen des Werkzeugs in der Aufnahmehülse begrenzen. Dabei kann das Werkzeug in der Umfangsrichtung unbeweglich positioniert sein oder ein gewisses Bewegungsspiel aufweisen, wenn ein entsprechend großer Abstand zwischen der Verrastungsnase und den Seitenrändern des Schlitzes vorhanden ist.

Durch einen Freistich im Fußbereich der Verrastungsfläche der Verrastungsnase läßt sich auch bei einer kleinen Verrastungsnase mit einer kleinen Verrastungsfläche eine sichere Verrastung erreichen.

Zum Lösen des Werkzeugs ist ein hohlkegelförmiges Druckstück vorgesehen, das mit seinem Umfangsrand gegen die Einführungsfläche der wenigstens einen Verrastungsnase drückbar ist, wobei letztere gegen die elastische Rückstellkraft radial nach innen in eine Freigabestellung bewegt werden. Das Druckstück ist auf der dem Werkzeug abgewandten Seite des Handstücks von außen zugänglich. Es kann so in das Handstück integriert sein, daß es gegen den Werkzeugschaft in eine Lösestellung schiebbar ist und durch eine elastische Rückstellkraft in seine Ausgangsstellung zurückbewegt wird. Das Druckstück kann jedoch auch ein als separates loses Bauteil ausgebildeter Stift sein, der durch ein auf der dem Werkzeug gegenüberliegenden Seite angeordnetes Loch in das Handstück einsteckbar und gegen den Halteschaft manuell bewegbar ist sowie nach der Lösung des Werkzeugs wieder herausziehbar ist.

In weiteren Unteransprüchen sind Merkmale enthalten, die die elastische Rückstellung der wenigstens einen Verrastungsnase verbessern, einfach und kostengünstig herstellbare Ausgestaltungen gewährleisten und ein handhabungsfreundliches Verbinden und Lösen des Werkzeugs mit dem Handstück ermöglichen.

Nachfolgend werden vorteilhafte Ausgestaltungen der Erfindung anhand eines in Zeichnungen dargestellten Ausführungsbeispiels näher beschrieben. Es zeigt
- Fig. 1: ein medizinisches oder dentalmedizinisches Behandlungsinstrument mit einem erfindungsgemäßen Handstück in der Seitenansicht;
- Fig. 2: das vordere Ende des Behandlungsinstruments bzw. Handstücks im axialen Schnitt;
- Fig. 3: ein Werkzeug für das Behandlungsinstrument bzw. Handstück, teilweise axial geschnitten, in einer Seitenansicht;
- Fig. 4: das Werkzeug in einer um 90° verdrehten Seitenansicht;
- Fig. 5: das Werkzeug in einer um 90° weiter verdrehten Seitenansicht.

Die Hauptteile des in seiner Gesamtheit mit 1 bezeichneten Behandlungsinstruments sind ein stabförmiges Handstück 2, das durch eine Schnellkupplung 3 in Form einer Steckkupplung, vorzugsweise eine Steck/Drehkupplung, mit einem Anschlußteil 4 lösbar verbunden ist, das durch eine lösbare Verbindungsvorrichtung 5, z. B. eine Schraubverbindung, mit einem flexiblen Versorgungsschlauch 6 verbindbar ist, der sich von einem nicht dargestellten Steuergerät eines medizinischen oder dentalmedizinischen Behandlungsstuhls erstreckt und mit dem Steuergerät vorzugsweise lösbar verbunden ist.

Im vorderen Endbereich des Handstücks 2 ist eine Aufnahmehülse 7 als Teil einer Fixiervorrichtung F für ein andeutungsweise dargestelltes Werkzeug 10 drehbar gelagert. Die Aufnahmehülse 7 steht in Antriebsverbindung mit einem Antrieb, mit einer sich längs durch die Griffhülse 2a des Handstücks erstreckenden und darin drehbar gelagerten Antriebswelle 8 und einem im Handstückkopf angeordneten Getriebe 9, das die Drehbewegung der Antriebswelle 8 in eine in die Längsrichtung des Werkzeugs 10 gerichtete Hin- und Herbewegung umwandelt.

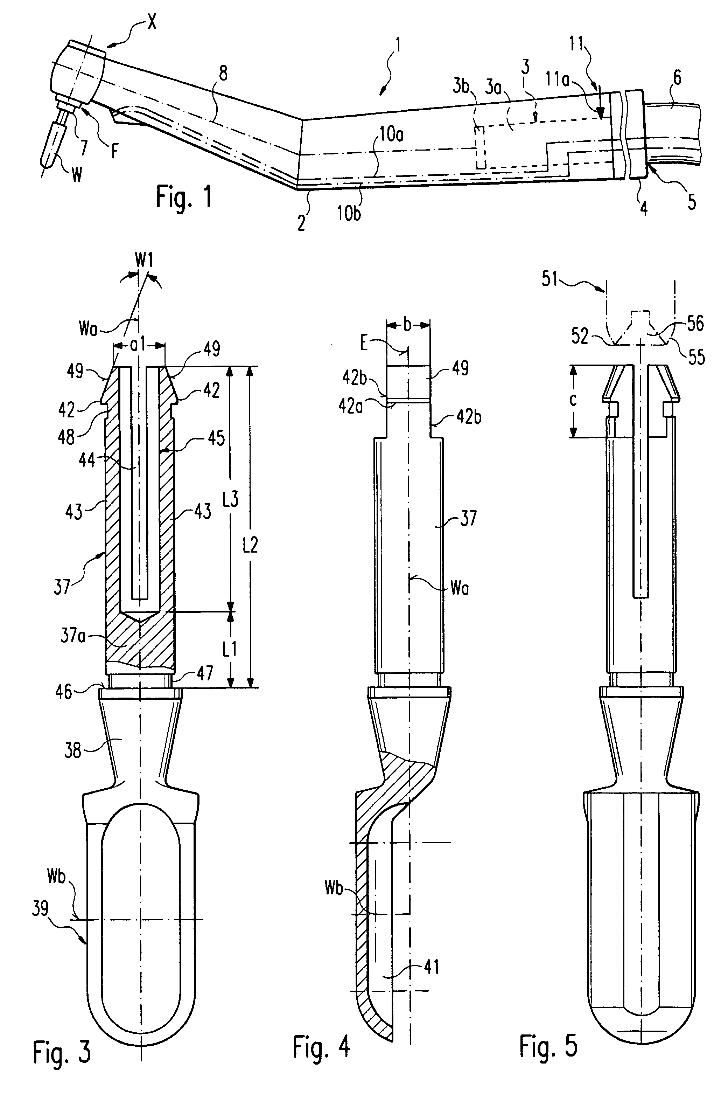

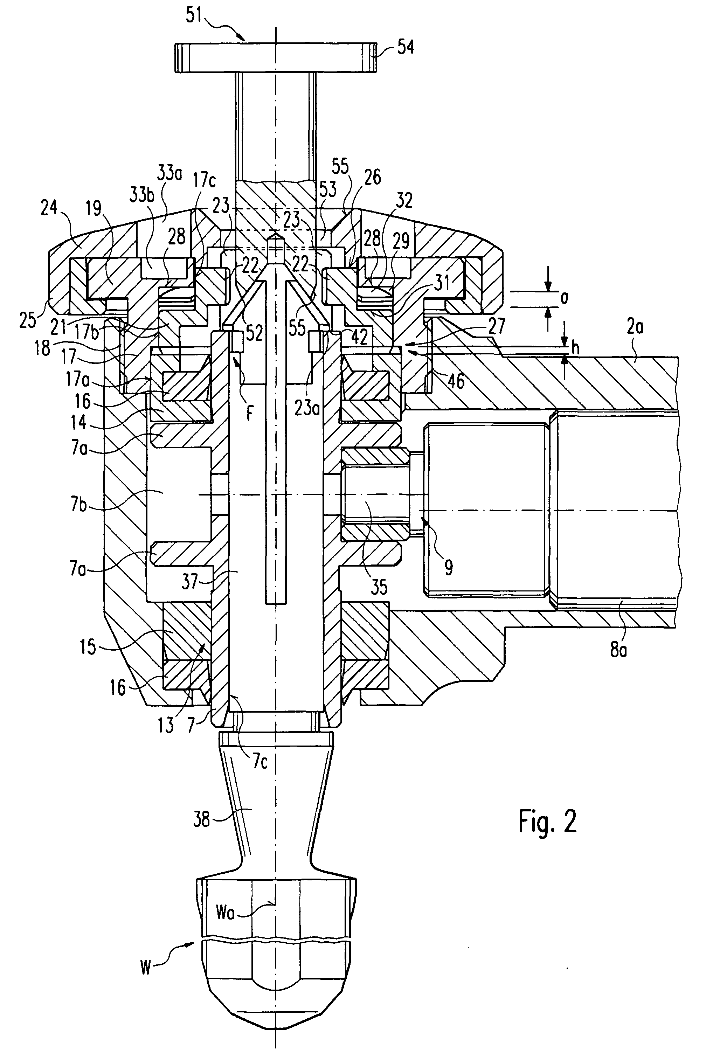

Das Handstück 2 ist durch die Griffhülse gebildet, die sich gerade (nicht dargestellt) oder winkelförmig im Sinne eines sogenannten Winkelhandstücks (Fig. 1) oder bogenförmig gekrümmt erstrecken kann. Durch die Steck/Drehkupplung wird die Handhabbarkeit des Handstücks 2 wesentlich verbessert, weil aufgrund der freien Drehbarkeit im Bereich der Steck/Drehkupplung das Anschlußteil 4 an Drehbewegungen des Handstücks 2 während der Behandlung nicht teilzunehmen braucht. Die Steck/Drehkupplung weist einen hohlzylindrischen Kupplungszapfen 3a am einen Kupplungsteil und eine den Kupplungszapfen 3a mit geringem Bewegungsspiel aufnehmende Kupplungsausnehmung 3b auf. Bei der vorliegenden Ausgestaltung erstreckt sich der Kupplungszapfen 3a vom Anschlußteil 4 nach vorne und die Kupplungsausnehmung 3b ist im hinteren Endbereich des Handstücks 2 angeordnet. Es sind eine oder mehrere Medienleitungen, z. B. zwei Medienleitungen 10a, 10b für Luft und Wasser, vorgesehen, die sich axial durch den Kupplungszapfen 3a erstrecken, die zylindrische Trennfuge zwischen dem Kupplungszapfen 3a und der Kupplungsausnehmung 3b in einem axialen Abstand voneinander radial durchsetzen und sich Z-förmig in der Griffhülse fortsetzen, wobei sie im vorderen Endbereich auf die Behandlungsstelle ausgerichtet aus der Griffhülse austreten. Die wenigstens eine Medienleitung 10a, 10b durchsetzt die Trennfuge im Bereich einer Ringnut im Kupplungszapfen 3a oder in der Kupplungsausnehmung 3b. Die Ringnut ist axial auf beiden Seiten abgedichtet. Z. B. durch in Aufnahmenuten angeordneten O-Ringen. Hierdurch ist der Mediendurchgang in jeder Drehstellung der Steck/Drehkupplung gewährleistet. Zur axialen lösbaren Positionierung in der Kupplungsstellung dient eine manuell überdrückbare Verrastungsvorrichtung 11 mit einem quer bewegbar gelagerten Verrastungselement 11a, das in einer Ringausnehmung in der Außenmantelfläche des Kupplungszapfens 3a oder in der Innenmantelfläche der Kupplungsausnehmung 3b angeordnet ist und durch eine elastische Federkraft in eine im jeweils gegenüberliegend anderen Teil angeordnete Verrastungsausnehmung so nachgiebig hineingedrückt wird, daß das Verrastungselement 11a ausfedern kann und die Verrastungsvorrichtung 11 durch eine axial gerichtete und manuell leicht aufbringbare Zug- und Druckkraft überdrückbar ist. Hierdurch ist bei Aufrechterhaltung des Mediendurchgangs ein Drehen der Kupplung um 360° möglich.

Ein Antriebsmotor ist im Anschlußteil 4 angeordnet (nicht dargestellt). Die Antriebswelle 8 steht im gekuppelten Zustand der Steck/Drehkupplung durch eine Steckkupplung mit dem Antriebsmotor in Antriebsverbindung, die beim Trennen des Handstücks 2 vom Anschlußteil 4 selbsttätig gelöst wird.

Wie Fig. 2 zeigt, weist die Griffhülse 2a in ihrem vorderen Längsbereich eine axiale Lagerbohrung 12 auf, die nach vorne in eine zu ihr etwa rechtwinklig durchgehend verlaufende und in Stufen gefertigte Lagerbohrung 13 mündet. In der Lagerbohrung 13 ist die hohlzylindrische Aufnahmehülse 7 in ihrer Längsrichtung, d.h. quer zum Handstück 2, um ein paar Millimeter hin und her verschiebbar und drehbar gelagert. In der zylindrischen Bohrung 8 der Aufnahmehülse 7 ist das Bearbeitungswerkzeug 10 mit einem längs seiner Mittelachse 10a angeordneten zylindrischen Halteschaft von 10 ist von längs oszillierender Arbeitsweise, wobei es sich beim vorliegenden Ausführungsbeispiel um ein im Querschnitt flaches bzw. schwertförmiges Werkzeug 10 im Sinne einer Feile handelt, mit dessen Breit- und/oder Schmalseiten bei seiner Hubbewegung spanabhebend gearbeitet werden kann oder auch - je nach Feinheit - geglättet bzw. poliert werden kann.

Die Aufnahmehülse 7 weist in ihrem mittleren Bereich zwei einen axialen Abstand voneinander aufweisende Flansche 7a auf, zwischen denen eine Umfangsnut 7b rechteckigen Querschnitts gebildet ist. In ihren zu beiden Seiten der Flansche 7a gelegenen Endbereichen ist die Aufnahmehülse 7 in Gleitlagerringen 14, 15 gelagert, die im Bereich der zu beiden Seiten vorhandenen Lagerbohrungsabschnitte angeordnet sind bzw. diese bilden, wobei an der Außenseite jedes Gleitlagerringes 14, 15 jeweils ein Dichtungsring 16 mit einer axial nach außen weisenden Abstreiflippe angeordnet ist.

Das werkzeugferne Gleitlager ist mit seinem Gleitlagerring 14 in einer Buchse 17 angeordnet, die in einen äußeren stufenförmig vergrößerten Endabschnitt der Lagerbohrung 6 eingeschraubt ist (Gewinde 18). Die Buchse 17 weist auch einen axial äußeren Drehflansch 19 und eine dreistufige Bohrung auf, mit einem inneren Bohrungsabschnitt 17a, in dem der Gleitlagerring 14 fest eingesetzt ist, einem mittleren Bohrungsabschnitt 17b, in dem ein Mitnehmerring 21 mit Bewegungsspiel drehbar gelagert ist und einen äußeren Bohrungsabschnitt 17c. Der Mitnehmerring 21 weist einen Z-förmigen Querschnitt auf, wobei am äußeren, verjüngten Ringabschnitt 21a innenseitig einander diametral gegenüberliegende Mitnehmerzapfen 22 einstückig angeformt sind, die mit geringem Bewegungsspiel in einander diametral gegenüberliegende radiale Schlitze 23 der Aufnahmehülse 7 einfassen, wobei die Schlitze 23 axial endseitig ausmünden.

Auf dem Flansch 19 ist ein Drehflansch 24 mit geringem Bewegungsspiel drehbar gelagert, der axial außenseitig vom Flansch 19 angeordnet ist und mit seinem äußeren Rand 25 den Flansch 19 C-förmig über- und hintergreift, wodurch der Drehflansch 24 in axialer und radialer Richtung am Flansch 19 drehbar gehalten ist. Am Außenumfang weist der Drehflansch 24 zur Verbesserung einer Griffestigkeit Rillen, Stege oder eine Rändelung auf. In der Innenwandung des Drehflansches 24 oder des Flansches 19 können einander diametral gegenüberliegend axial und radial angeordnete Schlitze 26 vorgesehen, in die mit geringem Bewegungsspiel die axial vorspringenden Mitnehmerzapfen 22 mit ihren radial äußeren Bereichen einfassen. Hierdurch ist eine formschlüssig wirksame Drehverbindung zwischen dem Drehflansch 24 und dem Mitnehmerring 21 geschaffen.

Der Gleitlagerring 14 und der Mitnehmerring 21 liegen mit Stirnflächen einander gegenüber, und sie greifen mit jeweils einer an diesen Stirnflächen angeordneten Verzahnung 27 ineinander. Es ist eine solche Verzahnung vorgesehen, die durch Drehen des einen Teils relativ zum anderen Teil überdrückbar ist. Dies ist durch schräge Flanken der Verzahnung 27 möglich, wobei die Neigung der schrägen Flanken an das maximale Drehmoment, das die Verzahnung 27 übertragen soll, so angepaßt ist, daß bei Überschreitung dieses Drehmoments der Mitnehmerring 21 axial nach außen aus dem Zahneingriff herausgedrückt wird. Hierzu ist dem Mitnehmerring 21 ein axialer Bewegungsfreiraum zwischen dem Gleitlagerring 14 und der inneren Stufenfläche 28 der Buchse 17 vorgesehen. Dieser Bewegungsfreiraum 29 ist axial durch die Stufenfläche 28 der Buchse 17 und die Stufenfläche 31 des Mitnehmerrings 21 begrenzt. Der Abstand a dazwischen ist größer bemessen, als die Höhe h der Verzahnung 27 und der Höhe von einer oder mehreren im Bewegungsfreiraum 29 angeordneten Wellfedern 32, die den Mitnehmerring 21 axial in den Verzahnungseingriff elastisch vorspannen.

Im Drehflansch 24 und im Flansch 19 sind zwei einander diametral gegenüberliegende, jeweils miteinander in Flucht bringbare axiale Bohrungen 33a, 33b vorgesehen. Durch die äußeren Bohrungen 33a im Drehflansch 24 hindurch können die Zapfen eines nicht dargestellten Schraubschlüssels in die Bohrungen 33b eingesteckt werden zum Zweck des Ein- oder Ausschraubens der Buchse 17. Der Flansch 19 und der Drehflansch 24 sind fest miteinander verbunden und bilden somit ein gemeinsames Drehteil.

Um die axiale Hubbewegung der Aufnahmehülse 7 zu gewährleisten, bedarf es zu beiden Seiten der Flansche 7a eines Freiraums und einer entsprechenden Länge der Schlitze 23, damit die inneren Mitnehmerzapfen 22 nicht gegen die inneren Schlitzenden stoßen.

Für den Antrieb der Aufnahmehülse 7 ist am vorderen Ende des Antriebswellenzuges des Handstücks 2 ein exzentrischer zylindrischer Antriebszapfen 35 vorgesehen, der in die Umfangsnut 7b mit geringem Bewegungsspiel einfaßt. Beim vorliegenden Ausführungsbeispiel ist ein den Antriebszapfen 35 tragender Antriebswellenabschnitt 8 mit einem Kegelradgetriebe im Knickbereich des Handstücks 2 vorgesehen, und in der Griffhülse in Wälz- oder Gleitlagern drehbar gelagert.

Beim vorbeschriebenen Handstückkopf 1 können wahlweise Drehstellungen des Werkzeugs 9 bezüglich des Handstückkopfs 1 eingestellt werden und zwar unabhängig von der jeweiligen Hubstellung der Aufnahmehülse 7. Die Dreheinstellung kann in kleinen Stufen erfolgen, was durch die verhältnismäßig kleine Teilung der Verzahnung 27 gewährleistet ist. Die Verzahnung 27 bildet eine lösbare Kupplung 46 zwischen dem Mitnehmerring 21 und dem Gleitlagerring 14, die bei Überschreitung eines vorbestimmten Drehmoments selbsttätig öffnet und bei Unterschreitung des Drehmomentes selbsttätig wieder schließt. Hierdurch ist es möglich, durch Drehen des Drehflansches 24 in die eine oder andere Drehrichtung eine gewünschte Drehstellung des Werkzeugs 9 einzustellen, wobei die Verzahnung 26 überdreht wird, d.h., wie ein Klinkensperrtrieb im Leerlauf wirkt. Wenn andererseits am Werkzeug 9 ein vorbestimmtes Drehmoment überschritten wird, folgt die Aufnahmehülse 7 dieser Drehbeanspruchung selbsttätig, wobei die Verzahnung 27 ebenfalls überdrückt wird. Hierdurch sind Druckbeanspruchungen oder Beschädigungen der Zähne ausgeschlossen, die auf vom Handstück 2 bei der Handhabung ausgehenden Hebelwirkungen beruhen.

Es ist nicht erforderlich, daß die Verzahnung 27 an beiden Verzahnungsteilen vollständig ausgebildet sein muß. Die Funktion ist dann gewährleistet, wenn an einem Teil ein vollständiger Zahnkranz und am anderen Teil theoretisch nur ein, vorzugsweise zwei oder mehrere auf dem Umfang verteilt angeordnete Zähne vorgesehen sind. Bei der vorliegenden Ausgestaltung sind drei Zähne gleichmäßig verteilt angeordnet und zwar vorzugsweise am Mitnehmerring 21. Am Gleitlagerring 14 ist dagegen ein vollständiger Zahnkranz mit schräge Flanken aufweisenden entsprechenden Zähnen vorgesehen.

Je nach Art der Zahnbehandlung kann es vorteilhaft sein, mit einem Werkzeug zu arbeiten, daß bis zu einem vorbestimmbaren Drehmoment nicht drehbar ist oder mit einem Werkzeug zu arbeiten, daß um seine Längsmittelachse leicht oder frei drehbar ist. Letzteres wird durch die Ausgestaltung ermöglicht, bei der dem Handstückkopf 1 eine axial wirksame Verstelleinrichtung zugeordnet ist, in deren einen Verstellposition die Kupplung 46 eingerückt und somit das Werkzeug begrenzt drehfest ist, und in deren anderer Verstellposition die Einrück-Vorspannung der Kupplung 46 verringert oder ganz aufgehoben und somit das Werkzeug 9 leicht oder frei drehbar ist. Die Verstelleinrichtung enthält ein Verstellglied, mit dem das eine, hier das drehfest mit der Aufnahmehülse 7 verbundene Kupplungsteil ausrückbar und in der ausgerückten Stellung fixierbar ist, oder mit dem die Einrück-Vorspannung der Kupplung 46 verringert oder ganz aufgehoben werden kann.

Das Werkzeug 10 besteht aus dem axialen Halteschaft 37, der vorzugsweise zylindrisch ausgebildet ist, und einem sich daran unmittelbar anschließenden oder durch einen Verbindungsschaft 38 verbundenen Arbeitsschaft 39, der wenigstens eine seitliche abrasive Arbeitsfläche und eine unrunde, vorzugsweise abgeflachte Querschnittsform aufweist. Der Arbeitsschaft 39 kann die Form eines Löffels oder einer Schaufel aufweisen, die bezüglich der Werkzeugachse 10 außermittig versetzt ist, siehe Fig. 4. Der konkave Hohlraum der Löffel- bzw. Schaufelform ist mit 41 bezeichnet. Die konvexe Seitenfläche der Löffel- bzw. Schaufelform ist abrasiv, z. B. diamantiert.

Im freien Endbereich des Halteschaftes 37 sind eine oder mehrere, beim vorliegenden Ausführungsbeispiel zwei einander gegenüberliegend quer abstehende Verrastungsnasen 42 angeordnet, die aus einer dargestellten Verrastungsstellung gegen eine elastische Rückstellkraft in eine radial nach innen verlagerte Freigabestellung bewegbar sind und selbsttätig in ihre Verrastungsstellung radial ausfedern. Die Verrastungsfläche 42a der Verrastungsnase 42 erstreckt sich in einer Radialebene.

Die wenigstens eine Verrastungsnase 42 ist vorzugsweise an einem sich axparallel erstreckenden Federarm 43 angeordnet, der sich von einer Halteschaftbasis 37a zum freien Ende des Halteschaftes 37 hin erstreckt. Die axiale Länge L1 der Halteschaftbasis 37a ist vorzugsweise geringer als die Hälfte der Länge L2 des Halteschaftes 37. Vorzugsweise beträgt die Länge L1 ¼ der Länge L2. Die Länge L3 der Federarme 43 beträgt somit mehr als die Hälfte der Länge L2 des Halteschaftes 37, vorzugsweise etwa ¾ der Länge L2.

Der oder die Verrastungsarme 43 ist bzw. sind durch einen sich in der Achsrichtung des Werkzeugs 10 erstreckenden Schlitz 44 vom Halteschaft 37 getrennt. Wenn zwei Federarme 43 oder Verrastungsnasen 42 einander gegenüberliegend angeordnet sind, ist ein gemeinsamer Schlitz 44 in mittiger Position vorgesehen. Um die Elastizität bzw. die elastische Biegbarkeit der Federarme 43 zu verbessern, sind diese durch eine innenseitige Materialausnehmung verjüngt. Hierdurch bleibt der äußere Mantelflächenabschnitt der Federarme 43 unverändert, der vorzugsweise ein zylindrischer Mantelflächenabschnitt des zylindrischen Halteschaftes 37 bzw. der Halteschaftbasis 37a ist. Beim vorliegenden Ausführungsbeispiel ist die innenseitige Verjüngung der Federarme 43 durch ein koaxiales Loch 45, insbesondere eine Bohrung, gebildet, die sich vom freien Ende des Halteschaftes 37 her in den Halteschaft 37 erstreckt, vorzugsweise bis in die Nähe des Grundes des Schlitzes 44, wobei sie den Schlitzgrund auch überragen kann, wie es Fig. 3 zeigt.

Die Einstecktiefe des Halteschaftes 37 ist durch eine an seinem Fußende angeordnete Anschlagschulter 46 begrenzt, die beim vorliegenden Ausführungsbeispiel durch eine Verdickung des Verbindungsschaftes 38 gebildet ist, der sich zum Arbeitsschaft 39 hin vorzugsweise kegelförmig verjüngt. An der Anschlagschulter 46 weist der Halteschaft 37 einen Freistich 47 in Form einer durch eine Ringnut gebildeten Verjüngung auf, der im Funktionsbetrieb eine sichere Anlage der Anschlagschulter 46 an der Stirnfläche der Aufnahmehülse 7 gewährleistet, da die Randkante der Einstecköffnung der Aufnahmehülse bei eingestecktem Werkzeug 10 freiliegt.

Aus den gleichen Gründen ist auch an der bzw. den Verrastungsnasen 42 ein Freistich 48 in der Mantelfläche des zugehörigen Federarmes 43 angeordnet. Die Rückenfläche der Verrastungsnase 42 ist eine in Richtung auf das freie Ende des Halteschaftes 37 konvergente Einführungsfläche 49, die in axialer Richtung schräg oder gerundet geformt sein kann und vorzugsweise eine Kegelabschnittfläche ist. Die Einführungsfläche 49 endet stirnseitig mit einer Querschnittsabmessung a, die kleiner ist als die Einstecköffnung der Aufnahmehülse 7. Hierdurch ist ein handhabungsfreundliches Einstecken des Halteschaftes 37 in die Aufnahmehülse 7 gewährleistet, wobei die wenigstens eine von der Mantelfläche des Halteschaftes 37 quer abstehende Verrastungsnase 42 gemeinsam mit dem Federarm 43 auf ein Querschnittsmaß einfedert, das gleich oder kleiner ist als die Innenquerschnittsabmessung der Aufnahmehülse 7. In der eingesteckten Anschlagstellung federt die Verrastungsnase 42 hinter dem Querrand 23a des Schlitzes 23 selbsttätig aus, wobei sie verrastet.

Die wenigstens eine Verrastungsnase ist seitlich auf ein solches Quermaß b abgeflacht, daß sie in den Schlitz 23 paßt. Beim vorliegenden Ausführungsbeispiel, bei dem der vorzugsweise rechteckförmige Schlitz mittig angeordnet ist, ist die Verrastungsnase 42 beidseitig abgeflacht, so daß sie mit Bewegungsspiel zwischen die seitlichen Begrenzungsränder des Schlitzes 23 paßt. Die mit 42b bezeichneten seitlichen Abflachungsflächen erstrecken sich vorzugsweise parallel zur Bewegungsebene der Verrastungsnase 42. Die axiale Abmessung c der Abflachungsflächen 42b kann größer bemessen sein, als die axiale Abmessung der wenigstens einen Verrastungsnase 42, wie es Fig. 4 und 5 zeigen.

Zum Fixieren des Werkzeugs 10 wird der Halteschaft 37 in die Aufnahmehülse 7 bis zum Anschlag 46 eingesteckt und manuell gedreht, bis die die wenigstens eine Verrastungsnase in den Schlitz 23 einschnappt.

Zum Lösen des Werkzeugs 10 ist es erforderlich, die wenigstens eine Verrastungsnase 42 in ihre Freigabestellung einzufedern. Dies kann durch ein ringförmiges Druckglied 51 erfolgen, das aus einer koaxial neben dem freien Ende des Halteschaftes 37 angeordneten Ausgangsstellung gegen den Halteschaft 37 in eine Ausschubstellung verschiebbar ist. Während dieser Ausschubbewegung trifft der ringförmige Rand 52, dessen Innenabmessung größer ist als die Querschnittsabmessung a, gegen die Einführungsflächen 49. Aufgrund der Divergenz der Einführungsflächen 49 und eines sich dabei ergebenden Keiltriebes wird die wenigstens eine Verrastungsnase 42 radial nach innen in ihre Freigabestellung gedrängt, in der das Werkzeug 10 manuell aus der Aufnahmehülse 7 herausgezogen werden kann.

Bei dem Druckglied 51 kann es sich um ein im Handstück 2 koaxial verschiebbar gelagertes Teil handeln, das z. B. durch eine Rückstellfeder in seine Ausgangsstellung vorgespannt ist. Das Druckglied 51 kann jedoch auch ein separates stiftförmiges Bauteil sein, das durch ein im wesentlichen koaxial zur Aufnahmehülse 7 angeordnetes Durchführungsloch 53 von der dem Werkzeug 10 abgewandten Seite her in das Handstück 2 einsteckbar und gegen den Halteschaft 37 schiebbar ist. Ein solches Druckglied 51 kann einen vorzugsweise flachen Kopf 54 aufweisen, um die Druckbelastung am Bedienungsfinger der Bedienungshand, mit dem das Druckglied 51 in seine Ausschubstellung bewegt wird, gering zu halten.

Insbesondere bei einem als separates Bauteil ausgebildeten Druckglied ist es vorteilhaft, den Außenrand des Durchführungslochs 53 und/oder den Außenrand des ringförmigen Randes 52 im Sinne einer schrägen oder gerundeten Einführungsfläche 55 auszubilden, wodurch das Einstecken des ringförmigen Randes in das Durchführungsloch 53 erleichtert wird. Das Druckglied 51 kann durch einen runden Stift in Form einer Hülse oder aus Formmaterial gebildet sein. Im letzteren Fall kann der ringförmige Rand 52 durch eine z. B. hohlkegelförmige Ausnehmung 56 im freien Stirnende des Stiftes gebildet sein. Die wenigstens eine Einführungsfläche 49 schließt mit der Mittelachse der Ausnehmungshülse 7 bzw. des Werkzeugs 10 einen spitzen W von etwa 15 bis 60°, insbesondere etwa 30°, ein. Den gleichen Winkel kann auch die Mantelfläche der Ausnehmung 56 aufweisen.

Beim vorliegenden Ausführungsbeispiel erstreckt sich die bezüglich der Längsmittelachse 10a quer verlaufende Längsachse 10b der länglichen Querschnittsform des Arbeitschaftes 39 in der Bewegungsebene E der wenigstens einen Verrastungsnase 42 oder parallel dazu. Im ersteren Fall ist der Arbeitsabschnitt 39 im wesentlichen mittig angeordnet. Im zweiten Fall ist der Arbeitsschaft 39 bezüglich der Bewegungsebene E und zugleich Mittelebene quer versetzt, wie es Fig. 4 zeigt. Im Rahmen der Erfindung ist es jedoch auch möglich, die Richtung der quer verlaufenden Längsachse 10b und/oder die Versatzrichtung in anderen Querrichtungen anzuordnen.

Das Werkzeug 10 und die Aufnahmehülse 7 bestehen vorzugsweise aus korrosionfestem Metall, insbesondere legiertem Stahl.

## Patentansprüche

1. Medizinisches oder dentalmedizinisches Handstück (2)
mit einem Werkzeug (10),
wobei das Handstück (2), in seinem vorderen Endbereich eine Aufnahmehülse (7) aufweist, in die ein zylindrischer Halteschaft (37) des Werkzeugs (10) einsteckbar und darin fixierbar ist,
die durch einen Antrieb in ihrer Achsrichtung hin und her verschiebbar gelagert ist
und die in einem axialen Abstand von einer Einstecköffnung wenigstens eine Hinterschneidung an ihrer Innenwand aufweist,
wobei der Halteschaft (37) in einem axialen Abstand von einem seine Einsteckbewegung begrenzenden Anschlag (46)
wenigstens eine radial abstehende und elastisch einfederbare Verrastungsnase (42) aufweist, die den Rand der Hinterschneidung hintergreift,
**dadurch gekennzeichnet,**
**dass** die Hinterschneidung durch einen mittigen ersten Schlitz (23) in der Aufnahmehülse (7) gebildet ist
und die Breite der Verrastungsnase (42) kleiner ist als die Innenquerabmessung der Aufnahmehülse (7).

2. Medizinisches oder dentalmedizinisches Werkzeug (10) für ein Handstück (2), das in seinem vorderen Endbereich eine Aufnahmehülse (7) aufweist,
in die ein zylindrischer Halteschaft (37) des Werkzeugs (10) einsteckbar und darin fixierbar ist,
die durch einen Antrieb in ihrer Achsrichtung hin und her verschiebbar gelagert ist
und die in einem axialen Abstand von einer Einstecköffnung wenigstens eine Hinterschneidung an ihrer Innenwand aufweist,
wobei der Halteschaft (37) in einem axialen Abstand von einem seine Einsteckbewegung begrenzenden Anschlag (46)
wenigstens eine radial abstehende und elastisch einfederbare Verrastungsnase (42) aufweist,
**dadurch gekennzeichnet,**
**dass** die Breite (b) der Verrastungsnase (42) kleiner ist als die Querschnittsabmessung des Halteschaftes (37).

3. Handstück oder Werkzeug nach einem der vorherigen Ansprüche,
**dadurch gekennzeichnet,**
**dass** die Rückenfläche der Verrastungsnase (42) durch eine konvergente Einführungsfläche (49) gebildet ist.

4. Handstück nach Anspruch 1 oder 3,
**dadurch gekennzeichnet,**
**dass** der Schlitz (23) beide einander gegenüberliegenden Wandungen der Aufnahmehülse (7) durchsetzt.

5. Handstück oder Werkzeug nach einem der vorherigen Ansprüche,
**dadurch gekennzeichnet,**
**dass** mehrere Verrastungsnasen (42), vorzugsweise zwei Verrastungsnasen (42), auf dem Umfang verteilt oder einander gegenüberliegend angeordnet sind.

6. Handstück oder Werkzeug nach einem der vorherigen Ansprüche,
**dadurch gekennzeichnet,**
**dass** die Verrastungsnasen (42) jeweils an einem sich in der Längsrichtung des Halteschaftes (37), insbesondere achsparallel, erstreckenden Federarm (43) angeordnet sind.

7. Handstück oder Werkzeug nach Anspruch 6,
**dadurch gekennzeichnet,**
**dass** die Federarme (43) durch einen vorzugsweise mittigen Schlitz (44) im Halteschaft (37) gebildet sind.

8. Handstück oder Werkzeug nach einem der vorherigen Ansprüche,
**dadurch gekennzeichnet,**
**dass** die wenigstens eine Verrastungsnase (42) im freien Endbereich des Halteschaftes (37) angeordnet ist.

9. Handstück oder Werkzeug nach einem der Ansprüche 6 bis 8,
**dadurch gekennzeichnet,**
**dass** der wenigstens eine Federarm (43) an seiner Innenseite verjüngt ist.

10. Handstück oder Werkzeug nach Anspruch 9,
**dadurch gekennzeichnet,**
**dass** die Verjüngung durch ein koaxiales Loch (45) im Halteschaft (37) gebildet ist.

11. Handstück oder Werkzeug nach einem der Ansprüche 6 bis 10,
**dadurch gekennzeichnet,**
**dass** die Länge (L3) des wenigstens einen Federarms (43) größer ist als die Hälfte der Länge (L2) des Halteschaftes (37).

12. Handstück oder Werkzeug nach einem der vorherigen Ansprüche,
**dadurch gekennzeichnet,**
**dass** im Fußbereich des Halteschaftes (37) ein die Einsteckbewegung des Werkzeugs (10) begrenzender Anschlag (46) vorgesehen ist.

13. Handstück oder Werkzeug nach einem der vorherigen Ansprüche,
**dadurch gekennzeichnet,**
**dass** an der wenigstens einen Verrastungsnase (42) und/oder am Anschlag (46) ein Freistich (47, 48) vorgesehen ist.

14. Handstück oder Werkzeug nach einem der vorherigen Ansprüche,
**dadurch gekennzeichnet,**
**dass** der Arbeitsschaft (39) eine längliche Querschnittsform aufweist und die sich quer erstreckende Längsachse (10b) der Querschnittsform sich in der Bewegungsebene (E) der wenigstens einen Verrastungsnase (42) oder parallel dazu erstreckt.

15. Handstück oder Werkzeug nach einem der vorherigen Ansprüche,
**dadurch gekennzeichnet,**
**dass** der Arbeitsschaft (39) die Form eines Löffels oder einer Schaufel aufweist.

## Claims

1. Medical or dental medical handpiece (2) having a tool (10)
the handpiece (2) having in its forward end region a receiving sleeve (7) into which a cylindrical holding shaft (37) of the tool (10) can be inserted and fixed therein
which is mounted to be displaceable back and forth in its axial direction by means of a drive
and which has at its inner wall, at an axial spacing from an insertion opening, at least one undercut,
the holding shaft (37) having, at an axial spacing from a stop (47) limiting its insertion movement,
at least one radially upstanding and elastically spring retractable latching nose (42) which engages behind the edge of the undercut,
**characterised in that**,
the undercut is formed by means of a middle first slit (23) in the receiving sleeve (7)
and the width of the latching nose (42) is smaller than the inner transverse dimension of the receiving sleeve (7).

2. Medical or dental medical tool (10) for a handpiece (2),
which has in its forward end region a receiving sleeve (7)
into which a cylindrical holding shaft (37) of the tool (10) can be inserted and fixed therein,
which is mounted to be displaceable back and forth in its axial direction by means of a drive
and which has at its inner wall, at an axial
spacing from an insertion opening, at least one undercut,
the holding shaft (37) having, at an axial spacing from a stop (47) limiting its insertion movement,
at least one radially upstanding and elastically spring retractable latching nose (42),
**characterised in that**,
the width (b) of the latching nose (42) is smaller than the transverse dimension of the holding shaft (37).

3. Handpiece or tool according to any preceding claim,
**characterised in that**,
the back surface of the latching nose (42) is formed by a convergent lead-in surface (49).

4. Handpiece according to claim 1 or 3,
**characterised in that**,
the slit (23) passes through both walls of the receiving sleeve (7) lying opposite to one another.

5. Handpiece or tool according to any preceding claim,
**characterised in that**,
a plurality of latching noses (42), preferably two latching noses (42), are arranged distributed on the circumference or lying opposite one another.

6. Handpiece or tool according to any preceding claim,
**characterised in that**,
the latching noses (42) are arranged in each case on a spring arm (43) extending in the longitudinal direction of the holding shaft (37), in particular axis-parallel.

7. Handpiece or tool according to claim 6,
**characterised in that**,
the spring arms (43) are formed by means of a preferably middle slit (44) in the holding shaft (37).

8. Handpiece or tool according to any preceding claim,
**characterised in that**,
that at least one latching nose (42) is arranged in the free end region of the holding shaft (37).

9. Handpiece or tool according to any of claims 6 to 8,
**characterised in that**,
the at least one spring arm (43) is tapered at its inner side.

10. Handpiece or tool according to claim 9,
**characterised in that**,
the tapering is formed by means of a coaxial hole (45) in the holding shaft (37).

11. Handpiece or tool according to any of claims 6 to 10,
**characterised in that**,
the length (L3) of the at least one spring arm (43) is greater than half the length (L2) of the holding shaft (37).

12. Handpiece or tool according to any preceding claim,
**characterised in that**,
in the foot region of the holding shaft (37) there is provided a stop (46) limiting the insertion movement of the tool (10).

13. Handpiece or tool according to any preceding claim,
**characterised in that**,
an undercut (47, 48) is provided on the at least one latching nose (42) and/or on the stop (46).

14. Handpiece or tool according to any preceding claim,
**characterised in that**,
the working shaft (39) has an elongate cross-sectional form and the transversely extending longitudinal axis (10b) of the cross-sectional form extends in the plane of movement (E) of the at least one latching nose (42), or extends parallel thereto.

15. Handpiece or tool according to any preceding claim,
**characterised in that**,
the working shaft (39) has the form or a spoon or of a shovel.

## Revendications

1. Pièce à main (2) médicale ou médico-dentaire,
comportant un outil (10),
la pièce à main (2) présentant dans sa zone d'extrémité avant une douille de réception (7) dans laquelle on peut insérer et fixer un axe-support cylindrique (37) de l'outil (10),
qui est montée de façon à pouvoir coulisser en va-et-vient parallèlement à son axe au moyen d'un système d'entraînement,
et dont la paroi interne présente, décalé axialement par rapport à une ouverture d'insertion, au moins un logement,
l'axe-support (37) présentant, décalé axialement par rapport à une butée (46) limitant son mouvement d'insertion :
au moins un ergot d'enclenchement (42) en saillie radiale et à effacement élastique, qui s'agrippe derrière le bord du logement,
**caractérisée en ce que**
le logement est formé par une première rainure médiane (23) dans la douille de réception (7),
et la largeur de l'ergot d'enclenchement (42) est inférieure à la dimension transversale interne de la douille de réception (7).

2. Outil (10) médical ou médico-dentaire pour une pièce à main (2),
qui présente dans sa zone d'extrémité avant une douille de réception (7),
dans laquelle on peut insérer et fixer un axe-support cylindrique (37) de l'outil (10),
qui est montée de façon à pouvoir coulisser en va-et-vient parallèlement à son axe au moyen d'un système d'entraînement,
et dont la paroi interne présente, décalé axialement par rapport à une ouverture d'insertion, au moins un logement,
l'axe-support (37) présentant, décalé axialement par rapport à une butée (46) limitant son mouvement d'insertion :
au moins un ergot d'enclenchement (42) en saillie radiale et à effacement élastique,
**caractérisé en ce que**
la largeur (b) de l'ergot d'enclenchement (42) est inférieure à la dimension transversale de l'axe-support (37).

3. Pièce à main ou outil selon l'une des revendications précédentes,
**caractérisé(e) en ce que** la surface arrière de l'ergot d'enclenchement (42) est formée par une surface d'introduction convergente (49).

4. Pièce à main selon la revendication 1 ou 3,
**caractérisée en ce que** la rainure (23) traverse les deux parois, opposées l'une à l'autre, de la douille de réception (7).

5. Pièce à main ou outil selon l'une des revendications précédentes,
**caractérisé(e) en ce que** plusieurs ergots d'enclenchement (42), de préférence deux ergots d'enclenchement (42), sont agencés sur la périphérie de manière répartie ou l'un en face de l'autre.

6. Pièce à main ou outil selon l'une des revendications précédentes,
**caractérisé(e) en ce que** les ergots d'enclenchement (42) sont agencés chacun sur un bras élastique (43) s'étendant dans la direction longitudinale de l'axe-support (37), de préférence parallèlement à l'axe.

7. Pièce à main ou outil selon la revendication 6,
**caractérisé(e) en ce que** les bras élastiques (43) sont formés par une fente (44) de préférence médiane dans l'axe-support (37).

8. Pièce à main ou outil selon l'une des revendications précédentes,
**caractérisé(e) en ce que** ledit au moins un ergot d'enclenchement (42) est agencé dans la zone d'extrémité libre de l'axe-support (37).

9. Pièce à main ou outil selon l'une des revendications 6 à 8,
**caractérisé(e) en ce que** ledit au moins un bras élastique (43) est rétréci sur sa face intérieure.

10. Pièce à main ou outil selon la revendication 9,
**caractérisé(e) en ce que** le rétrécissement est formé par un trou coaxial (45) dans l'axe-support (37).

11. Pièce à main ou outil selon l'une des revendications 6 à 10,
**caractérisé(e) en ce que** la longueur (L3) dudit au moins un bras élastique (43) est supérieure à la moitié de la longueur (L2) de l'axe-support (37).

12. Pièce à main ou outil selon l'une des revendications précédentes,
**caractérisé(e) en ce qu**'une butée limitant le mouvement d'insertion de l'outil (10) est prévue à la base de l'axe-support (37).

13. Pièce à main ou outil selon l'une des revendications précédentes,
**caractérisé(e) en ce qu**'une rainure de dégagement (47, 48) est prévue sur ledit au moins un ergot d'enclenchement (42) et/ou sur la butée (46).

14. Pièce à main ou outil selon l'une des revendications précédentes,
**caractérisé(e) en ce que** le fût de travail (39) présente une forme de section transversale allongée et l'axe longitudinal (10b), s'étendant transversalement, de la forme de section transversale, s'étend dans le plan de mouvement (E) dudit au moins un ergot d'enclenchement (42) ou parallèlement à celui-ci.

15. Pièce à main ou outil selon l'une des revendications précédentes,
**caractérisé(e) en ce que** le fût de travail (39) présente la forme d'une cuiller ou d'une pelle.
